Europäisches Patentamt

(19) European Patent Office

Office européen des brevets

(11) EP 0 527 498 B1

(12) **EUROPEAN PATENT SPECIFICATION**

(45) Date of publication and mention
of the grant of the patent:
**02.04.1997 Bulletin 1997/14**

(51) Int. Cl.⁶: $A61B\ 1/015$, $A61B\ 1/273$

(21) Application number: **92113840.0**

(22) Date of filing: **07.01.1988**

(54) **Body cavity pressure adjusting device for endoscope**

Körperhöhlendruckeinstellsystem für ein Endoskop

Dispositif d'ajustement de pression dans une cavité du corps pour un endoscope

(84) Designated Contracting States:
**DE FR GB**

(30) Priority: **09.01.1987 JP 3066/87**
            **26.06.1987 JP 158931/87**
            **17.07.1987 JP 179696/87**

(43) Date of publication of application:
**17.02.1993 Bulletin 1993/07**

(62) Document number(s) of the earlier application(s) in
accordance with Art. 76 EPC:
**88100123.4 / 0 278 217**

(73) Proprietor: **ASAHI KOGAKU KOGYO KABUSHIKI
KAISHA**
**Tokyo 174 (JP)**

(72) Inventors:
   • **Doi, Yuzuru,**
     **c/o Asahi Kogaku Kogyo K. K.**
     **Tokyo 174 (JP)**
   • **Honda, Ryoji,**
     **c/o Asahi Kogaku Kogyo K. K.**
     **Tokyo 174 (JP)**
   • **Shirai, Masami,**
     **c/o Asahi Kogaku Kogyo K. K.**
     **Tokyo 174 (JP)**

(74) Representative: **Schaumburg, Thoenes & Thurn**
   **Mauerkircherstrasse 31**
   **81679 München (DE)**

(56) References cited:
   **EP-A- 0 078 035**        **EP-A- 0 186 762**
   **WO-A-82/03545**        **DE-A- 3 716 230**
   **US-A- 3 730 645**

**Description**

This invention relates to a body cavity pressure adjusting device according to the first part of claim 1 and 2, respectively. A pressure adjusting device of this kind is known from WO-A-82 3545.

In a medical examination or a treatment with an endoscope, air is supplied into a body cavity such as the stomach in order to spread the wall of the body cavity or in order to clean the objective lens of the endoscope.

In general, a body cavity internal organ such as the stomach or the intestines automatically contracts itself. Therefore, in a medical examination of a body cavity with an endocsope, air is supplied into the body cavity through an air supplying pipe line built in the endoscope so as to inflate the body cavity widely enough for an operator to observe it sufficiently.

If the stomach is excessively filled with air, however, then the gastric wall may be so greatly spread that the small blood vessels are broken, which will result in the stomach hemorrhage. As the stomach is greatly strained, the patient may belch. Thus, the supplying of air into the stomach inflicts pain on the patient. Since the gastric wall may be excessively spread, as described above, it is difficult to accurately detect delicate changes in the mucous membranes, which in turn makes it difficult to form a correct diagnosis on or to give the patient a proper medical treatment for gastritis by utilizing an endoscope.

In those circumstances, a device for automatically adjusting body cavity pressure has been in demand in order to eliminate such inconvenience.

In a conventional device, a sucking operation period is fixed. Consequently, an amount of the suction is sometimes too much and sometimes too little according to the capacity of the body cavity in which an endoscope is inserted.

An object of the invention is to provide a body cavity pressure adjusting device for an endoscope wherein the body cavity pressure is able to be automatically adjusted to a level that an operator wishes, and high utilities are imparted.

This problem is solved by a body cavity pressure adjusting device according to claim 1 or 2. Further embodiments of the invention are described in the subclaims.

The present invention may be more fully understood from the description of a preferred embodiment of the invention set forth below, together with the accompanying drawings, in which:

Figure 1 is a block diagramm showing an entire structure of one embodiment of this invention,

Figure 2 is an outside perspective view of a device of the embodiment,

Figure 3 is a perspective view in which the suction pipe is shown as bending downwardly,

Figures 4, 5, 6 and 7 are flow charts showing programs stored in a memory of a microcomputer,

Figure 8 is a chart of the table stored in the memory of the microcomputer,

Figure 9 is a graph showing the experimental result obtained by measuring adjustment conditions of the body cavity pressure by utilizing the device of the embodiment,

Figure 10 is a side view showing one example of piping in the device,

Figure 11 is a perspective view of an electromagnetic valve, and

Figure 12 is a perspective view of a tube unit.

DESCRIPTION OF THE PREFERRED EMBODIMENT

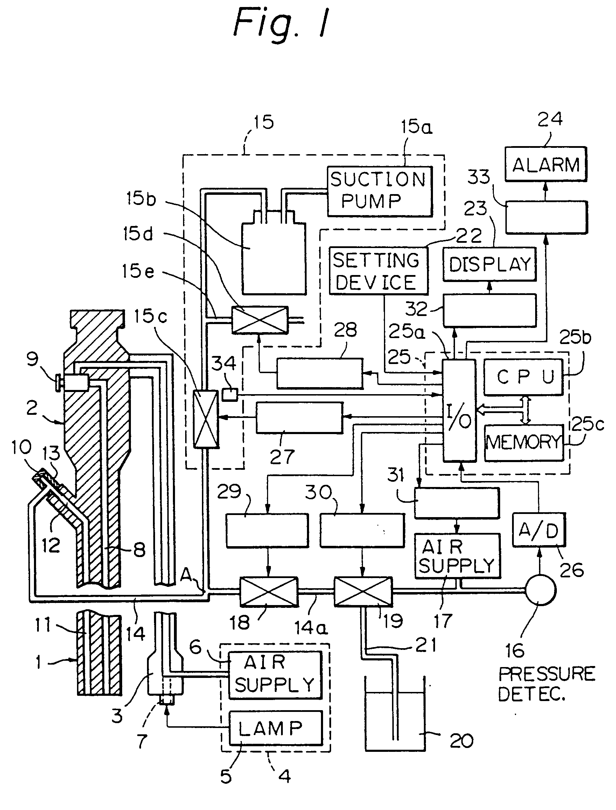

As shown in Figure 1, reference numerals 1, 2 and 3 respectively represent an inserted part of an endoscope which is inserted into a body cavity, an operation part and a connector connected to an external light source unit 4.

Provided inside the light source unit 4 are an illumination lamp 5 and an air pump as an air supplying device 6. Illumination light is injected into an illuminating light guide 7 provided in the endoscope from the illumination light 5, and gas such as air is delivered into an air supplying pipe 8 formed through the endoscope from the air supplying device 6. The air supplying pipe 8 extends from the connector 3 to the inserted part 1 through the operation part 2 and is open at the tip of the inserted part 1. On its way the air supplying pipe 8 has a control valve 9 provided in the operation part 2 in such a way as to project therefrom.

This control valve 9 is well known to public. In a normal condition the air supplying pipe 8 is communicating with the atmosphere, whereby air sent into by the air supplying device 6 is discharged outside therefrom. Once the control valve 9 is pressed by a finger to cut off the communication with the atmosphere, the air is then supplied into the body cavity from the opening at the distal end of the inserted part 1. An electric switch can be provided so as to replace a control valve such as used in this embodiment. A device for supplying air continuously or a device for automatically controlling air supplying operation can be used as an air supplying device.

Reference numeral 11 is a forceps channel through which a medical tool such as a forceps is inserted. The channel is open at the distal end of the inserted part 1 and the proximal end of the channel is made to communicate with a forceps inserting port 12. Reference numeral 14 is a flexible suction tube made of polyurethane resin, silicone resin or the like and is releasably connected to the forceps inserting port 12 of the endoscope through a connecting clasp 13 connected to the mouth thereof in such a way as to communicate therewith. Therefore, if an endoscope has a forceps channel provided therein, it can be additionally provided with an automatic body cavity pressure adjustment function by utilizing the device of this invention. Provided on the connecting clasp 13 is a well known forceps plug 10 such as a rubber plug with a slit formed therein, through which a medical tool is enabled to be inserted into the forceps channel 11. When no medical tool is in the forceps channel 11, the forceps plug 10 is closed airtight.

The suction tube 14 is connected to an external suction device 15, and the suction tube 14 together with the forceps channel 11 forms a suction pipe whose end is open at the distal end of the inserted part 1. The suction device 15 comprises a suction pump 15a, a suction bottle 15b for storing what is sucked thereinto and a first and a second electromagnetic valve 15c, 15d. The first electromagnetic valve 15c is a valve of a type which is normally closed and the second electromagnetic valve 15d is a valve of a type which is normally opened. A valve opening and closing counter 34 for counting the number of opening and closing of the valve is provided on the first electromagnetic valve 15c, which is provided on the way of the suction tube 14.

The second electromagnetic valve 15d is provided on the way of a ventiduct 15e which is branched from the suction pipe on the way between the first electromagnetic valve 15c and the suction bottle 15b so as to be open to the atmosphere.

In this embodiment, therefore, with the suction pump 15a continuously running, air is taken into from the ventiduct 15e by closing the first electromagnetic valve 15c in synchronism with opening the second electromagnetic valve 15d, and once the first electromagnetic valve 15c is opened in association with closing the second electromagnetic valve 15d, suction is enabled only through the suction tube 14. Sucking operation is thus controlled by association of the first and the second electromagnetic valve 15c and 15d.

Provided on the way of the suction pipe 14 so as to communicate therewith is a branch pipe 14a, at whose end a pressure detector 16 for detecting the pressure inside the suction tube 14 is provided. Therefore, if the suction pipe (the suction tube 14, the forceps channel 11) and the branch pipe 14a are not closed, the body cavity pressure is able to be detected through these pipes.

"A" indicates a branched portion. A second air supplying device 17 for supplying air into the suction tube 14 is provided on the way of the branch pipe 14a closer to the pressure detector 16. This second air supplying device 17 is, for instance, a small air supplying pump, which is adapted to continuously or intermittently supply a small amount of air (for instance, 30-70 ml per minute) into the suction tube 14.

Provided adjacent to the branched portion "A" is a third electromagnetic valve 18 of a type which is normally open, and a pipe control three-way electromagnetic valve 19 is provided between the third electromagnetic valve 18 and the pressure detector 16. A water tube 21 is provided in such a way as to communicate with a water bottle 20 for storing cleansing water or the like therein. Accordingly, controlling the three-way electromagnetic valve 19 enables the suction pipe 11 to communicate either with the pressure detector 16 or with the water tube 21 through the branched pipe 14a.

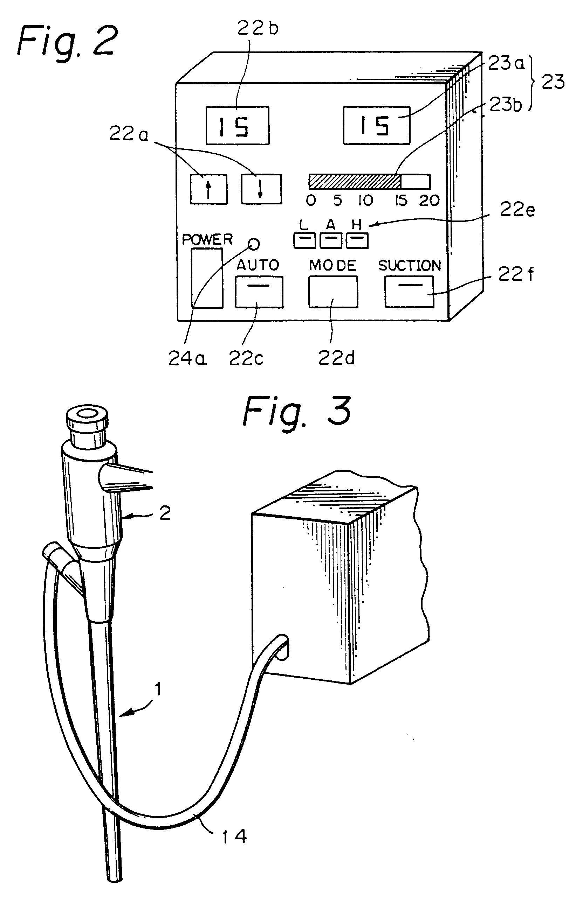

Reference numerals 22, 23 and 24 respectively represent a setting device for setting the body cavity pressure at a level an operator desires and also for setting an internal pressure adjustment mode, a pressure display for indicating the body cavity pressure detected or the like and an alarming device for providing an alarm when an unusual thing breaks out. These devices will be described hereinbelow with respect also to Figure 2 showing appearance of the device.

In Figure 2, reference numeral 22a represents pressure setting switches for setting the body cavity pressure at a level an operator desires, one of the two being pressed to increase a pressure value to be set and the other pressed to decrease a pressure value to be set. Foot-treadle type foot switches (not shown) having the same function as that of the pressure switches 22a can be additionally provided. Set pressure set by the switches is indicated on a set pressure display 22b.

Reference numeral 22c is a switch for turning on and off an automatic adjustment function for the body cavity pressure. When the switch 22c is on, the body cavity pressure is automatically adjusted, and when it is off, no automatic adjustment of the body cavity pressure is performed and a pressure detected by the pressure detector 16 is only indicated. Reference numeral 22d is a suction mode setting switch for setting a suction time of one sucking operation for the adjustment of the body cavity pressure. A condition (low, auto, high) set is indicated on a mode indicator lamp 22e. Reference numeral 22f represents a suction switch for controlling the three-way electromagnetic valve 19 so as to select sucking conditions from the water pipe 21. The setting switch 22 is composed of each of these switches 22a to 22f and the others.

A pressure display 23 is designed to indicate a pressure detected by the pressure detector 16 or the like and is composed of reference numeral 23a and 23b; reference numeral 23a is a digital display for digital indication and reference 23b is a bar graph display for analog indication. Reference numeral 24a is an alarm light and an alarm buzzer (not shown) adapted to operate in association with the alarm light 24a is installed inside the device.

In Figure 1, reference numeral 25 represents a microcomputer which comprises an input/output interface 25a, a central processing unit 25b and a memory 25c. The terminals of the input/output interface 25a are connected to output lines such as an output line from the setting device 22, an output line from the pressure detector 16 through an analog-digital (A/D) convertor 26 and an output line from the valve opening and closing counter 34. The output terminals of the input/output interface 25a are connected to each of the electromagnetic valves 15c, 15d, 18 and 19 through drive circuits 27, 28, 29 and 30, and also to the air supplying device 17, the pressure indicator 23 and the alarming device 24 through drive circuits 31, 32 and 33.

Operation of the embodiment will be described hereinafter.

After the inserted part 1 of the endoscope is inserted into a patient's body cavity, air is supplied by manually controlling the control valve 9 in the operation part 2 into the body cavity from the air supplying device 6 inside the light source unit 4 to thereby increase the body cavity pressure.

The connecting clasp 13 of the internal pressure adjusting device is connected to the forceps inserting port 12 in advance so as to automatically control and maintain the body cavity pressure below a pressure selectively set.

Even in this condition, a medical tool such as a forceps is able to be inserted into the forceps channel 11 through the forceps plug 10, so that the tool may be freely used therein. With the automatic adjustment on-off switch 22c set for "on", the most suitable pressure for a specific examination is set by utilizing the pressure setting switch 22a, and the pressure set is indicated on the set pressure display 22b.

In a normal condition wherein the suction switch 22f is off, the first electromagnetic valve 15c is close, the second electromagnetic valve 15d and the third electromagnetic valve 18 being open and the three-way electromagnetic valve 19 allowing the branch pipe 14a to communicate with the pressure detector 16. This communication enables the pressure detector 16 to detect the body cavity pressure through the suction tube 14 and the others, and an output signal from the pressure detector 16 is converted into a digital signal through the analog-digital convertor 26, the digital signal then being sent to the input/output interface 25a to be entered therein. The output signal from the input/output interface 25a orders the pressure display 23 (the digital display 23a and the bar graph display 23b) through the drive circuit 32 to indicate thereon the body pressure detected. The digital display 23a is adapted to normally indicate the detected pressure detected by the pressure detector 16 in real time, and when indication of the digital display 23a is made to be disable by the microcomputer 25, the indication immediately before execution of the order is designed to remain as indicated. With this display, when the suction device 15 is in operation, the pressure detected immediately before commencement of the sucking operation continues to be indicated on the display until the sucking operation is finished. The display, therefore, does not indicate a pressure which is widely different from the body cavity pressure.

In a normal condition, closure of the first electromagnetic valve 15c disconnects communication between the suction device 15 and the suction tube 14, whereby air is sucked only through the ventiduct 15e. The second air supplying device 17 supplies a small amount of air into the branch pipe 14a.

In CPU 25b, comparison between the set pressure set by the setting device 22 and the detected pressure detected by the pressure detector 16 is made. When the detected pressure becomes greater than the set pressure, an output signal from the input/output interface 25a orders indication of the digital display 23a to become disable, whereby indication thereon is fixed to the indicated value immediately before execution of the order. In synchronism with this operation, the second air supplying device 17 is made to stop supplying air, and the second and the third electromagnetic valve 15d, 18 are closed, while the first electromagnetic valve 15c is opened. But after a fixed period of time all relevant devices are designed to restore a normal condition thereof.

Therefore, during such a fixed period of time the suction tube is made to communicate with the suction device 15, whereby the air in the body cavity is sucked out through the forceps channel 11 to thereby reduce the body cavity pressure. The microcomputer 25 stores this fixed period of time during which this sucking (pressure reducing) operation is performed, and the sucking operation time is designed to change in accordance with a signal from the suction mode setting switch 22d. In other words, when the suction mode is set at the high mode, the pressure reducing operation is performed for a second, while it is performed for 0.2 second when set at the low mode. Accordingly, in a condition wherein change in an amount of air in the body cavity is very small, the operation time per one operation is set for a short period of time, while in a condition wherein air is continuously supplied into the body cavity, the operation time is set for a long period of time.

In contrast, when the suction mode is set at the auto mode, the pressure reducing operation is performed, respectively, for 0.3 second when the set pressure is less than 10 mmHg, for 0.5 second when the set pressure ranges from 10 mmHg to 15 mmHg, and for 0.7 second when the set pressure is more than 15 mmHg. Thus a suction amount by one operation of the suction device is automatically adjusted so as to be the most suitable for a set pressure, whereby the body cavity pressure is maintained stable all the time whether the body cavity pressure is set at a high or low value. With the device of this invention, as described above, the most suitable mode is able to be selected in accordance with a condition wherein an examination with an endoscope is performed.

In a case where blood or mucus clogs the forceps channel 11 or the suction tube 14 somewhere along the way thereof, the pressure detector 16 is caused not to detect the body cavity pressure. But when the suction tube 14 or the like is choked, a tiny amount of air supplied by the second air supplying device 17 increases the pressure inside the tube, and then the increasing pressure is eventually detected by the pressure detector 16, which starts the suction (pressure reducing) operation performing as described above, whereby the mucus or the like which is choking the suction tube 14 is automatically sucked and is discharged from the tube by the suction device 15. In synchronism with this operation, since the third electromagnetic valve 18 is closed, mucus or the like moving inside the suction tube 14 is prevented from entering into the pressure detector 16, whereby the pressure detector 16 is made to be free from influence of negative pressure caused from the suction. This prevents deterioration of the pressure detector and imparts a long life to it.

As illustrated in Figure 3, however, in a case where mucus or the like collects at a downwardly bending portion of the suction tube 14, one sucking (pressure reducing) operation is not enough to discharge the mucus from the bending portion, and once the sucking operation is finished, the mucus often returns to the bottom of the bending portion of the tube. In this case, the pressure detector 16 detects atmospheric pressure (0 mmHg) or a pressure less than that, when an output signal from the input/output interface 25a of the microcomputer 25 instructs the digital display 23a to indicate an indication which has nothing to do with a pressure value (e.g. "EE") to thereby show that the indicated value is nothing like the body cavity pressure. Accordingly, an operator can understand the situation from such an indication and then can take a suitable countermeasures.

At the same time, the sucking operation is repeated by increase in the pressure inside the tube caused by air supply from the second air supplying device 17. When the pressure detector 16 continues to detect atmospheric pressure or a pressure less than that during the sucking operation is repeated three times, the sucking operation is made to continuously perform for a long period of time (e.g. 1.5 seconds) by an output signal from the microcomputer 25 to thereby automatically discharge the mucus which collects at the bending portion of the suction pipe. Once the detected pressure exceeds atmospheric pressure, indication of the indicator and the suction time are designed to restore a normal condition thereof.

Alarm is raised when air is so excessively supplied into the body cavity that the sucking capacity of this device cannot afford to reduce the pressure properly. That is, when the detected pressure detected by the pressure detector 16 exceeds a predetermined pressure (e.g. 20 mmHg) a predetermined times (e.g. 3 times), an output signal from the microcomputer 25 turns on the alarm light 24a and at the same time the alarm buzzer goes off. The alarm is designed to be provided, as described above, only when the detected pressure still remains high after operation of the suction device has been performed a plural times in succession. Thus the alarm is given only when the body cavity pressure is unusually high.

Once the detected pressure decreases below a predetermined pressure, the alarm discontinues. While the automatic adjustment switch 22c is turned off, if the detected pressure exceeds a predetermined pressure continuously, for example, for more than two seconds, the alarm is given.

When the inside of the suction tube 14 and the branch pipe 14a is washed after an examination with the endoscope is finished, the connecting clasp 13 of the suction tube 14 is removed from the forceps inserting port 12 and is put into the water bottle 20. The tip of the water tube 21 is also put into the cleansing water in the water bottle 20. When the suction switch 22f is then turned on, an output signal of the microcomputer 25 changes the indication of the digital display 23a to, for example, an indication like "- -". In synchronism with this operation the first and the third electromagnetic valves 15c and 18 are opened, while the second electromagnetic valve 15d is closed. The three-way electromagnetic valve 19 is then controlled, for example, twice at an interval of 0.5 second to change its position so as to establish communication between the suction tube 14 and the water tube 21 through the branch pipe 14a. As the suction tube 14 is longer than the water tube 21, so does the suction tube 14 have a greater resistance therein than that of the water tube 21, while the three-way electromagnetic valve 19 is made to be open to the water tube 21, the cleansing water in the water bottle 20 is sucked out into the branch pipe 14a through the water tube 21 to thereby wash the inside thereof. Accordingly, sanitary problems caused from clogging of the branch pipe 14a or collection of filth therein are avoided, whereby the device may always be maintained properly for a normal and safe usage.

When the three-way electromagnetic valve 19 disconnects the cleansing water communication between the branch pipe 14a and the water tube 21, the cleansing water is then sucked into the suction tube 14 to thereby wash the inside thereof.

The tubes in the vicinity of each of the electromagnetic valves provided on the suction tube 14 and the branch pipe 14a are deteriorated by repetition of the sucking operation, in other words, repetition of opening and closing operation of the electromagnetic valves. In this invention, a tube replacement timing is designed to be advised by means of alarm. An output signal from the valve opening and closing counter 34 is made to enter into the microcomputer 25, and when the first electromagnetic valve 15c has performed the opening and closing operation, for instance, two hundred thousand times, an output signal from the microcomputer 25 turns on the alarm light 24a.

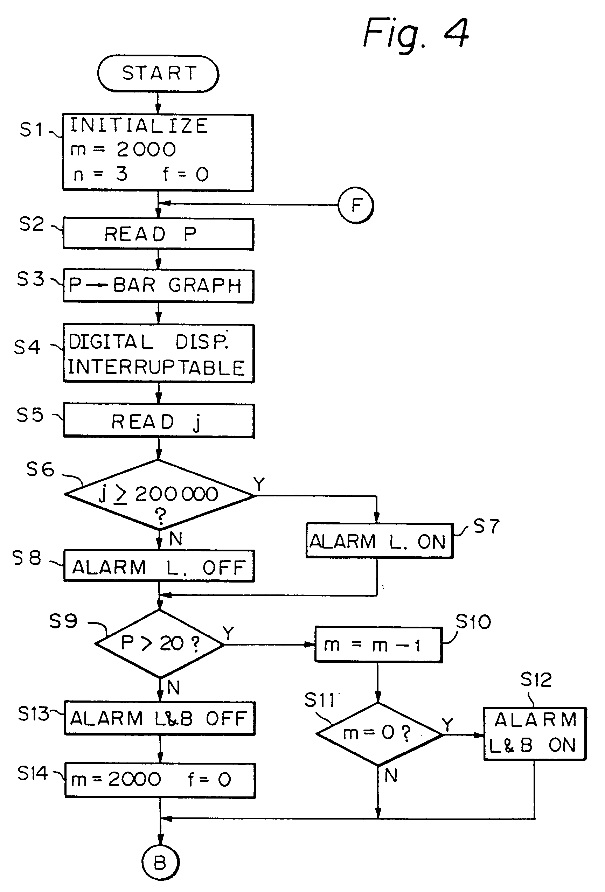

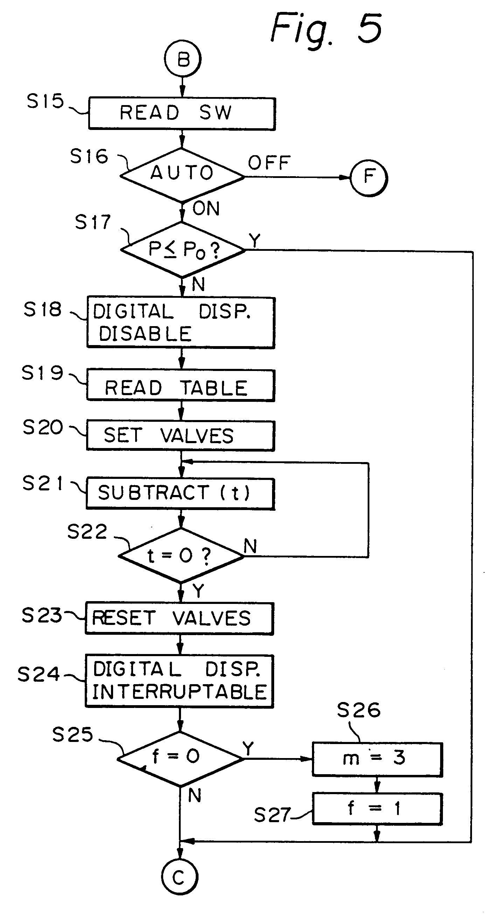

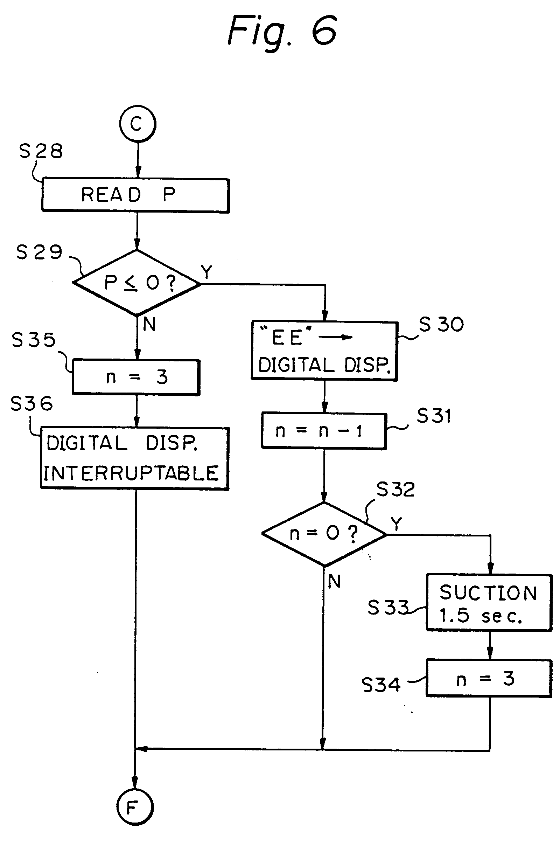

Figures 4 to 7 are flow charts showing programs stored in the memory 25c of the microcomputer 25, wherein "S" represents a step.

Figures 4 to 6 shows a program which is executed while the suction switch 22f is off, and in the case where there is no sucking operation occurring on the way, the program is executed about a thousand cycles per second on the average.

First, at a step S1, counters m, n and a flag f are initialized, wherein they are set, for instance, as follows: m = 2000, n = 3, f = 0. Then, at a step S2, the detected pressure is read, at a step S3, the detected pressure is indicated on the bar graph display 23b and at a step S4, indication of the digital display 23a is made to be interruptable.

At a step S5, the number of opening and closing "j" of the first electromagnetic valve 15c is read from the valve opening and closing counter 34, and at a step S6, whether or not the number of opening and closing "j" has reached the number for tube replacement (e.g. 200,000 times) is judged. When the number read has reached the number for tube replacement, at a step S7, the alarm light 24a is turned on and then the flow proceeds to a step S9. In the case where the number of opening and closing read at the step S6 is less than the number for tube replacement, the alarm light 24a is turned off at a step S8 and then the flow proceeds to the step S9.

Further, at the step S9, whether or not the detected pressure P exceeds 20 mmHg is judged, and when it exceeds 20 mmHg, at a step S10, an excessive pressure detecting counter m is set as follows: $m = m - 1$. At a step S11, if m is 0 (m = 0), at a step S12, the alarm light 24a and the alarm buzzer are turned on and then the flow proceeds to a step S15. In the case where m is not 0 at the step S11, then the flow proceeds directly to the step S15. When the detected pressure does not exceed 20 mmHg at the step S9, the alarm light 24a and the alarm buzzer are turned off at a step S13, and at a step S14, the excessive pressure detecting counter m and the flag f for the excessive pressure detecting counter are initialized again (m = 2,000, f = 0), the flow proceeding to the step S15.

At the step S15, status of the pressure setting switch and each of the other switches is read, and at a step S16, status of the automatic adjustment switch 22c is judged. When the automatic adjustment switch is off, the flow returns to the step S2, and when it is on, at a step S17, whether or not the detected pressure P is less than the set pressure Po is judged. When the detected pressure P is not less than the set pressure Po, indication of the digital display 23a is made to be disable to thereby lock the status of indication at a step S18, and at a step S19, the sucking operation time is selected from the table stored in the memory (ROM) in accordance with status of the mode setting switch 22d and the pressure setting switch 22a.

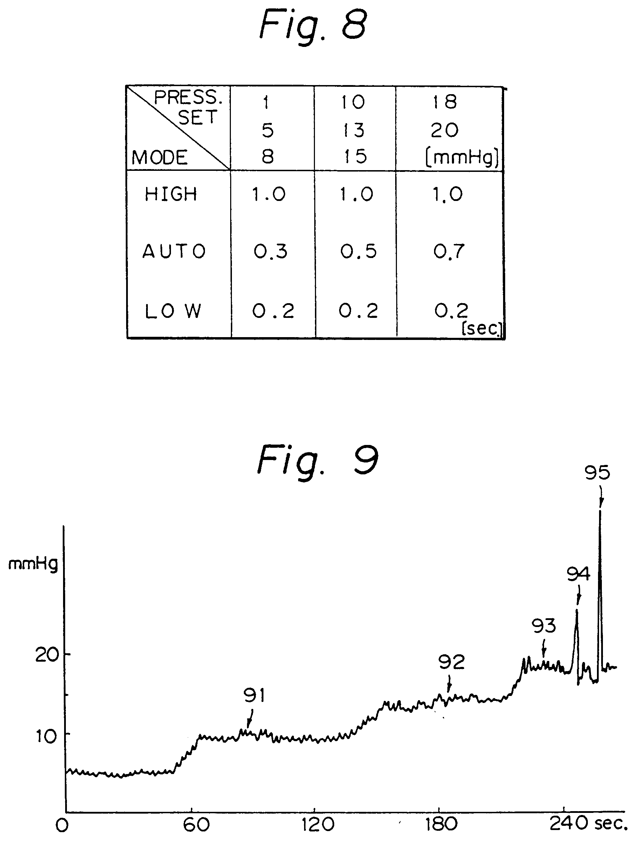

Figure 8 shows the table, wherein various suction times are given depending on the mode to be set and the pressure to be set. In this table, the suction time is fixed: 1 second for the high mode, 0.2 second for the low mode and for the auto mode, 0.3 second, 0.7 second and 0.5 second respectively when the set pressure is less than 10 mmHg, more than 15 mmHg and ranging from 10 to 15 mmHg.

At a step S20, the first, second and third electromagnetic valves 15c, 15d and 18 are set for the sucking (pressure reducing) operation. In other words, the first electromagnetic valve 15c is opened and the second and the third electromagnetic valves 15d, 18 are closed. At a step S21, the sucking operation time t is subtracted. When the remainder of the sucking operation time t is not 0 at a step S22, the flow returns to the step S21, when the remainder is 0, the first, second and third electromagnetic valves are made to restore a normal condition thereof at a step S23, and the digital display 23a is relieved from the disable status of indication, the flow proceeding to a step S25.

When the flag f for the excessive pressure detecting counter is 0 (f = 0) at the step S25, the excessive pressure detecting counter m is set for 3 (m = 3) at a step S26, and the flag is erected (f = 1), then the flow proceeding to a step S28. When the detected pressure P is less than the set pressure Po at the step S17, the flow then directly proceeds to the step S28.

At the step S28, the detected pressure P from the pressure detector 16 is read, and whether or not the detected pressure P is less than 0 mmHg is judged at a step S29. When the detected pressure P is less than 0 mmHg, the digital display 23a is made to indicate "EE" and such status of indication is locked at a step S30. Then, at a step S31, the detecting counter n is set as follows: $n = n - 1$. When n is not 0 at a step S32, the flow returns to the step S2, but when n is 0 at that step, the first to the third electromagnetic valves 15c, 15d and 18 are controlled so as to be put in the sucking (pressure reducing) condition for 1.5 seconds at a step S33. At a step S34, n is reset for 3 (n = 3), and the flow returns to the step S2. When the detected pressure is not less than 0 mmHg at the step S29, n is reset for 3 (n = 3) at a step S35, and the digital display 23a is relieved from the disable status of indication at a step S36, the flow then returning to the step S2.

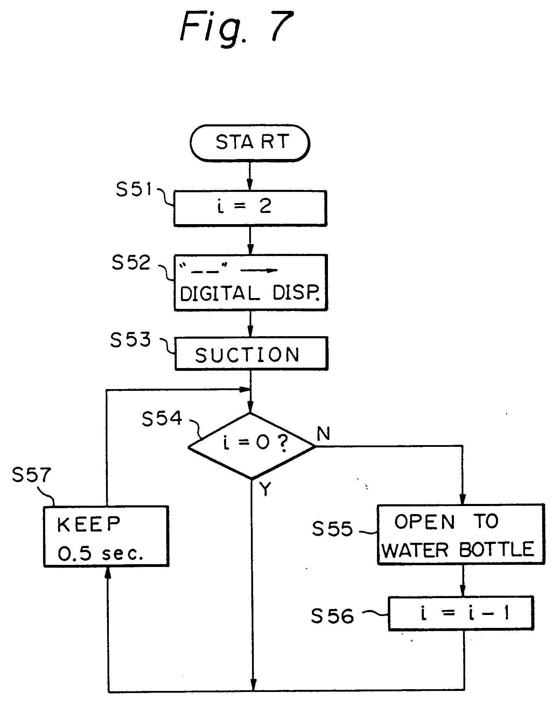

Figure 7 is a flow chart showing the program which is executed when the suction switch is on, wherein, first at a step S51, the counter i is initialized and i is set for 2 (i = 2). Then at a step S52, the digital display is made to indicate an indication such as "- -" and the indication status is locked, and at a step S53, the first to the third electromagnetic valves 15c, 15d, 18 are turned to the sucking condition. When i is not 0 at a step S54, then at a step S55 the three-way electromagnetic valve is made to be open to the water tube for 0.5 second, whereby the water tube 21 and the suction tube 14 are made to communicate with each other so that water is sucked from the water bottle 20. When the three-way electromagnetic valve restores a normal condition thereof after its 0.5 second opening, at a step S56 the counter i is set as follows: $i = i - 1$, and at a step S57, the flow is kept for 0.5 second and then returns to the step S54.

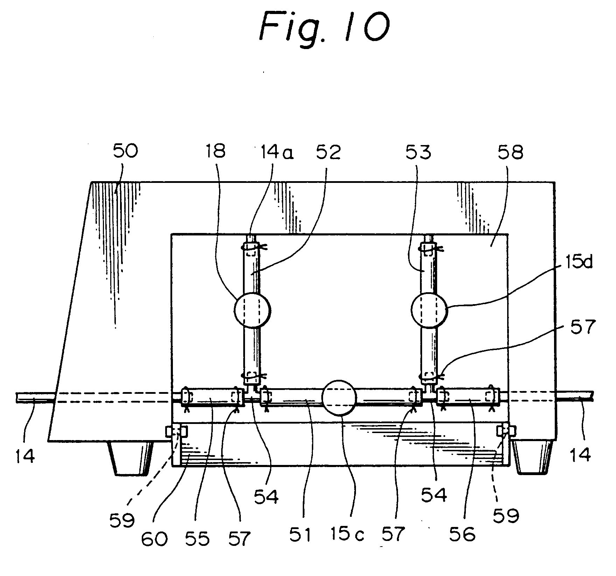

Figure 9 is a graph showing the data of the status of the body cavity pressure measured in an actual examination with an endoscope having the device of this embodiment provided.

In Figure 9, reference numerals 91, 92, and 93 respectively represent the status of the body cavity pressure when the set pressure is set for 10, 15, and 20 mmHg, and reference numerals 94 and 95 both represent momentary increases in the body cavity pressure caused by eructation. As is clear from this data, in an examination with an endoscope, the device of this embodiment can maintain a fixed body cavity pressure with high accuracy.

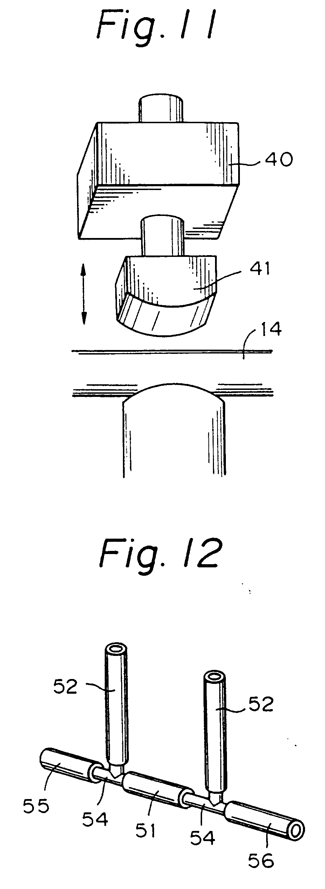

Figure 10 shows one example of piping for the device of this invention, wherein reference numeral 50 is a housing for the main body of the body cavity pressure adjusting device. The afore-mentioned first, second and third electromagnetic valve 15c, 15d and 18 are provided inside the housing. These electromagnetic valves are designed to close the pipes by, for instance as shown in Figure 11, collapsing the suction tube 14 from the sides thereof by a piston 41 directly connected to a magnetic device 40 in such a way as to move back and forth. In such a structure, mucus or the like to be sucked out is made to contact nothing but the inner surface of the suction tube 14, whereby washing or the like may be performed completely.

Tubes which pass through those electromagnetic valve portions are formed of thick and short silicone tubes 51, 52, 53 which are connected to each other through T-shaped pipes formed of synthetic resin 54, 54 which are in turn connected to the suction tube 14 on the way thereof through short silicone tubes 55, 56.

Mounting and removing of those tubes are designed to be performed, when needed, by removing spring clips 57 which are provided so as to fasten the connected portions. Therefore, not only is each of the tubes able to be replaced but also a whole of this portion is able to be replaced as a unit.

Out of these tubes, the tubes 51, 55, 56 which are positioned on the suction pipe on the way thereof are horizontally arranged, and the tubes 52, 53 forming branch pipes branched through the T-shaped pipes 54, 54 are arranged vertically upward. In such a construction, therefore, when filth or the like passes through the inside of the suction pipe, it is difficult for the filth to be allowed to enter into the branch pipes, and actually almost no filth enters thereinto.

The housing 50 has, as shown in Figure 10, a window 58 which is widely opened formed at the side of the branched portion wherein those respective tubes and electromagnetic valves are arranged, and the window 58 has a lid 60 which rotates about shafts 59 in such a way to open and close the window 58. Accordingly, once the lid 60 is opened, the branched portion can be observed, whereby the tubes can be checked on stains and leakages thereon, and at the same time replacement of the tubes can be performed. Instead, a transparent cover can be mounted on the window 58, or the lid 60 can be formed of transparent synthetic resin, so that the perimeter of the branched portion can always be observed from the outside.

While the invention has been described by reference to a specific embodiment chosen for illustrative purposes, it should be apparent that numerous modifications could be made thereto by those skilled in the art without departing from the basic concept and scope of the invention as defined in the claims.

## Claims

1. A body cavity pressure adjusting device for an endoscope (1) having a first channel (11) for receiving a medical tool therein, said device comprising a suction tube (14), suction means (15) connected to and communicating with said suction tube (14), pressure detecting means (16) for detecting pressure inside said suction tube (14), pressure setting means (22a) for setting a predetermined value for the body cavity pressure and suction control means (25) for controlling the operation of said suction means upon an output signal from said pressure detecting means (16) and an output signal from said pressure setting means (22a),
   **characterized** by

   - an air supply device (6) connectable to an air supply channel (8) in the endoscope (1) for supplying air into the body cavity through the endoscope,
   - one end of the suction tube (14) being connectable to the first channel (11),
   - the other end of the suction tube (14) being connected to the suction means (15) and to the pressure detecting means (16), for measuring the air pressure in the body cavity through the suction tube (14) and the first channel,

   wherein said suction control means (25) not only provides a signal ordering said suction means (15) to continuously operate for a fixed period of time but also includes a suction time setting means (22c, 22d) for setting said fixed period of time.

2. A body cavity pressure adjusting device for an endoscope (1) having a first channel (11) for receiving a medical tool therein, said device comprising a suction tube (14), suction means (15) connected to and communicating with said suction tube (14), pressure detecting means (16) for detecting pressure inside said suction tube (14), pressure setting means (22a) for setting a predetermined value for the body cavity pressure and suction control means (25) for controlling the operation of said suction means upon an output signal from said pressure detecting means (16) and an output signal from said pressure setting means (22a),
   **characterized** by

   - an air supply device (6) connectable to an air supply channel (8) in the endoscope (1) for supplying air into the body cavity through the endoscope,
   - one end of the suction tube (14) being connectable to the first channel (11),
   - the other end of the suction tube (14) being connected to the suction means (15) and to the pressure detecting means (16), for measuring the air pressure in the body cavity through the suction tube (14) and the first channel,

wherein said suction control means (25) provides a signal ordering said suction means (15) to continuously operate for a fixed period of time, said fixed period of time changing based upon a signal from said pressure setting means (22a).

## Patentansprüche

1. Körperhöhlendruckeinstellvorrichtung für ein Endoskop (1) mit einem ersten Kanal (11) zur Aufnahme eines medizinischen Werkzeuges in diesem, wobei die Vorrichtung ein Sauggrohr (14), eine Saugeinrichtung (15), die an das Saugrohr (14) angeschlossen ist und mit diesem in Verbindung steht, eine Druckerfassungseinrichtung (16) zum Erfassen eines Druckes in dem Saugrohr (14), eine Druckvorgabeeinrichtung (22a) zum Vorgeben eines vorbestimmten Wertes des Körperhöhlendruckes und eine Saugsteuereinrichtung (25) zum Steuern der Arbeitsweise der Saugeinrichtung in Abhängigkeit eines Ausgangssignals der Druckerfassungseinrichtung (16) sowie eines Ausgangssignales der Druckvorgabeeinrichtung (22a) umfaßt, **gekennzeichnet durch** eine Luftzufuhreinrichtung (6), die an einen Luftzufuhrkanal (8) in dem Endoskop (1) anschließbar ist, um Luft durch das Endoskop in die Körperhöhle einzuführen, wobei ein Ende des Saugrohres (14) mit dem ersten Kanal (11) verbindbar ist, wobei das andere Ende des Saugrohres (14) mit der Saugeinrichtung (15) und der Druckerfassungseinrichtung (16) verbunden ist, um den Luftdruck in der Körperhöhle durch das Saugrohr (14) und den ersten Kanal zu messen, und wobei die Saugsteuereinrichtung (25) nicht nur ein Signal liefert, welches die Saugeinrichtung (15) veranlaßt, über eine feste Zeitspanne kontinuierlich zu arbeiten, sondern auch eine Saugzeiteinstellvorrichtung (22c, 22d) zum Einstellen der festen Zeitspanne umfaßt.

2. Körperhöhlendruckeinstellvorrichtung für ein Endoskop (1) mit einem ersten Kanal (11) zur Aufnahme eines medizinischen Werkzeuges in diesem, wobei die Vorrichtung ein Sauggrohr (14), eine Saugeinrichtung (15), die an das Saugrohr (14) angeschlossen ist und mit diesem in Verbindung steht, eine Druckerfassungseinrichtung (16) zum Erfassen eines Druckes in dem Saugrohr (14), eine Druckvorgabeeinrichtung (22a) zum Vorgeben eines vorbestimmten Wertes des Körperhöhlendruckes und eine Saugsteuereinrichtung (25) zum Steuern der Arbeitsweise der Saugeinrichtung in Abhängigkeit eines Ausgangssignals der Druckerfassungseinrichtung (16) sowie eines Ausgangssignales der Druckvorgabeeinrichtung (22a) umfaßt, **gekennzeichnet durch**

eine Luftzufuhreinrichtung (6), die an einen Luftzufuhrkanal (8) in dem Endoskop (1) anschließbar ist, um Luft durch das Endoskop in die Körperhöhle einzuführen, wobei ein Ende des Saugrohres (14) mit dem ersten Kanal (11) verbindbar ist, wobei das andere Ende des Saugrohres (14) mit der Saugeinrichtung (15) und der Druckerfassungseinrichtung (16) verbunden ist, um den Luftdruck in der Körperhöhle durch das Saugrohr (14) und den ersten Kanal zu messen, und wobei die Saugsteuereinrichtung (25) ein Signal abgibt, welches die Saugeinrichtung (15) veranlaßt, für eine feste Zeitspanne kontinuierlich zu arbeiten, wobei die feste Zeitspanne in Abhängigkeit eines Signales von der Druckvorgabeeinrichtung (22a) variiert.

## Revendications

1. Dispositif de réglage de pression d'une cavité corporelle pour un endoscope (1) comportant un premier canal (11) pour y recevoir un outil médical, ledit dispositif comprenant un tube d'aspiration (14), un moyen d'aspiration (15) raccordé au et communiquant avec ledit tube d'aspiration (14), un moyen de détection de pression (16) pour détecter la pression à l'intérieur dudit tube d'aspiration (14), un moyen de réglage de pression (22a) pour régler une valeur prédéterminée de la pression de cavité corporelle et un moyen de commande d'aspiration (25) pour commander le fonctionnement dudit moyen d'aspiration d'après un signal de sortie provenant dudit moyen de détection de pression (16) et un signal de sortie provenant dudit moyen de réglage de pression (22a) ;

caractérisé :

par un dispositif de délivrance d'air (6) pouvant être raccordé à un canal de délivrance d'air (8) de l'endoscope (1) pour délivrer de l'air dans la cavité corporelle par l'intermédiaire de l'endoscope ;
en ce qu'une extrémité du tube d'aspiration (14) peut être raccordée au premier canal (11) ;
en ce que l'autre extrémité du tube d'aspiration (14) est raccordée au moyen d'aspiration (15) et au moyen de détection de pression (16), pour mesurer la pression d'air dans la cavité corporelle par l'intermédiaire du tube d'aspiration (14) et du premier canal ;

dans lequel ledit moyen de commande d'aspiration (25), non seulement délivre un signal ordonnant audit moyen d'aspiration (15) de fonctionner de manière continue pendant une période de temps fixe, mais encore comprend un moyen de réglage de temps d'aspiration (22c, 22d) pour régler ladite période de temps fixe.

2. Dispositif de réglage de pression d'une cavité corporelle pour un endoscope (1) comportant un premier canal (11) pour y recevoir un outil médical, ledit dispositif comprenant un tube d'aspiration (14), un moyen d'aspiration (15) raccordé au et communiquant avec ledit tube d'aspiration (14), un moyen de détection de pression (16) pour détecter la pression à l'intérieur dudit tube d'aspiration (14), un moyen de réglage de pression (22a) pour régler une valeur prédéterminée de la pression de cavité corporelle et un moyen de commande d'aspiration (25) pour commander le fonctionnement dudit moyen d'aspiration d'après un signal de sortie provenant dudit moyen de détection de pression (16) et un signal de sortie provenant dudit moyen de réglage de pression (22a) ;

    caractérisé :

    par un dispositif de délivrance d'air (6) pouvant être raccordé à un canal de délivrance d'air (8) de l'endoscope (1) pour délivrer de l'air dans la cavité corporelle par l'intermédiaire de l'endoscope ;

    en ce qu'une extrémité du tube d'aspiration (14) peut être raccordée au premier canal (11) ;

    en ce que l'autre extrémité du tube d'aspiration (14) est raccordée au moyen d'aspiration (15) et au moyen de détection de pression (16), pour mesurer la pression d'air dans la cavité corporelle par l'intermédiaire du tube d'aspiration (14) et du premier canal ;

    dans lequel ledit moyen de commande d'aspiration (25) délivre un signal ordonnant audit moyen d'aspiration (15) de fonctionner de manière continue pendant une période de temps fixe, ladite période de temps fixe variant en se basant sur un signal provenant dudit moyen de réglage de pression (22a).

# Fig. 1

Fig. 2

Fig. 3

# Fig. 4

Flowchart:

START

S1 — INITIALIZE
m = 2000
n = 3    f = 0

(F)

S2 — READ P

S3 — P → BAR GRAPH

S4 — DIGITAL DISP. INTERRUPTABLE

S5 — READ j

S6 — $j \geq 200\,000$ ?  — Y → S7 — ALARM L. ON

N

S8 — ALARM L. OFF

S9 — P > 20 ? — Y → S10 — m = m − 1

N

S11 — m = 0 ? — Y → S12 — ALARM L & B ON

N

S13 — ALARM L & B OFF

S14 — m = 2000    f = 0

(B)

## Fig. 5

```
                    (B)
                     │
                     ▼
S15 ─── READ SW
                     │
                     ▼
S16 ───  AUTO  ──OFF──→ (F)
                     │
                    ON
                     │
                     ▼
S17 ───  P ≤ P₀ ? ──Y──────────────┐
                     │              │
                     N              │
                     ▼              │
S18 ─── DIGITAL DISP.               │
        DISABLE                     │
                     │              │
                     ▼              │
S19 ─── READ TABLE                  │
                     │              │
                     ▼              │
S20 ─── SET VALVES                  │
                     │              │
                     ▼ ◄──────┐     │
S21 ─── SUBTRACT (t)          │     │
                     │        │     │
                     ▼        │     │
S22 ───  t = 0 ? ──N──────────┘     │
                     │              │
                     Y              │
                     ▼              │
S23 ─── RESET VALVES                │
                     │              │
                     ▼              │
S24 ─── DIGITAL DISP.               │
        INTERRUPTABLE               │
                     │        S26   │
                     ▼         ┌──────────┐
S25 ───  f = 0  ──Y──→         │  m = 3   │
                     │         └──────────┘
                     N              │
                     │         ┌──────────┐
                     │    S27 ─│  f = 1   │
                     │         └──────────┘
                     ▼ ◄────────────┘◄─────┘
                    (C)
```

$P \leq P_0$ ?

$t = 0$ ?

$f = 0$

$m = 3$

$f = 1$

# Fig. 6

S28 — READ P

S29 — P ≤ 0 ?

S35 — n = 3

S36 — DIGITAL DISP. INTERRUPTABLE

"E E" → DIGITAL DISP. — S30

n = n − 1 — S31

n = 0 ? — S32

SUCTION 1.5 sec. — S33

n = 3 — S34

# Fig. 7

```
                    ( START )
                        |
       S51 ──┐     ┌─────────┐
             └─────│  i = 2  │
                   └─────────┘
                        |
       S52 ──┐     ┌──────────────┐
             └─────│ "--" ──────→ │
                   │ DIGITAL DISP.│
                   └──────────────┘
                        |
       S53 ──┐     ┌──────────────┐
             └─────│   SUCTION    │
                   └──────────────┘
                        |
   ┌────────────────────┤
   |    S54 ──┐      ◇ i = 0 ?  ──N──┐
   |          └────   ◇              |
   |                   |Y            |
   |                                 |
 S57 ──┐   ┌─────────┐      S55 ──┐ ┌──────────────┐
       └───│  KEEP   │            └─│   OPEN  TO   │
          │ 0.5 sec. │             │ WATER BOTTLE │
          └─────────┘              └──────────────┘
   |          |                         |
   |          |           S56 ──┐ ┌──────────────┐
   |          |                 └─│  i = i - 1   │
   |          |                   └──────────────┘
   └──────────┴───────────────────────┘
```

# Fig. 8

| PRESS. SET / MODE | 1 5 8 | 10 13 15 | 18 20 [mmHg] |
|---|---|---|---|
| HIGH | 1.0 | 1.0 | 1.0 |
| AUTO | 0.3 | 0.5 | 0.7 |
| LOW | 0.2 | 0.2 | 0.2 [sec] |

# Fig. 9

# Fig. 10

# Fig. 11

# Fig. 12